# EUROPEAN PATENT APPLICATION

(11) **EP 3 360 562 A1**
(43) Date of publication of application: **15.08.2018**
(21) Application number: 16853897.3
(22) Date of filing: 06.10.2016
(51) Int. Cl.: A61K 36/575, A61K 31/36, A61K 31/34, A61K 8/97, A61K 8/49, A23L 33/105, A23L 33/10, A61Q 11/00

(54) **PHARMACEUTICAL COMPOSITION FOR PROMOTING PERIODONTAL TISSUE GROWTH AND FOR PREVENTION AND TREATMENT OF PERIODONTITIS, CONTAININGMAGNOLIA FLOWER**

(30) Priority: 07.10.2015 KR 20150140956; 01.04.2016 KR 20160040438
(71) Applicant: Choi, Bong Keun, Yongin-si, Gyeonggi-do 17153 (KR); Okinawa Research Center Co. Ltd., Okinawa 904-2234 (JP)
(72) Inventor: WOO, Je Tae, Uruma-shi Okinawa 904-2234 (JP)
(74) Representative: Office Freylinger
(86) International application number: PCT/KR2016/011171
(87) International publication number: WO 2017/061781

(57) **Abstract**

The present invention relates to a pharmaceutical composition comprising a *Magnolia flower* extract, a fraction thereof or the compound represented by formula 1 or formula 2 below as an active ingredient. Particularly, fargesin isolated from the said *Magnolia flower* extract and the fraction thereof can promote not only the growth of dental root but also the growth of periodontal tissue by forming alveolar bone in around teeth to inhibit periodontitis. Therefore, not only the fargesin but also the *Magnolia flower* extract comprising fargesin and the fraction thereof can be effectively used as an active ingredient for a pharmaceutical composition for promoting the growth of dental root and periodontal tissue and for preventing and treating periodontitis.

## Description

### BACKGROUND OF THE INVENTION

### 1. Field of the Invention

The present invention relates to a pharmaceutical composition for accelerating the growth of dental root or periodontal tissue, or preventing and treating periodontitis, which comprises a *Magnolia flower* extract, a fraction thereof or a compound isolated therefrom as an active ingredient.

### 2. Description of the Related Art

Periodontal tissue is a complicated organ containing both soft and hard tissues consisting of gingiva (gums), cementum, periodontal ligament and alveolar bone. The gingiva is a simple connective tissue involved in supporting and chewing process of teeth, whereas the periodontal ligament is a highly differentiated cellular connective tissue which is located between the teeth and the alveolar bone to support and attach the teeth to the jaw, to resist mechanical stresses in various directions such as masticatory force, and also to have sensory function. The periodontal ligament comprises various cells such as osteoblasts and osteoclasts on the alveolar bone side; fibroblasts, epithelial rests of Malassez, macrophages, undifferentiated mesenchymal cells, neural factors, vascular endothelial cells on the center of the periodontal ligament; and cementoblasts on the dental root side, and the periodontal ligament is known to be involved in restoration and regeneration of alveolar bone, dental root, and cementum.

The gingiva is a part of the tooth supporting tissue observed in the mouth, from which a disease (gingivitis) is developed. When the disease is worsen and spreads deeply into the supporting tissue, the periodontal ligament binding to the bone tissue surrounding the tooth root can be destroyed and also the alveolar bone covering the tooth can be destroyed, resulting in periodontal disease.

Periodontal disease is a kind of inflammation in the tooth supporting tissue caused by bacteria, which can be divided into gingivitis and periodontitis. The cause of this disease is the formation of dental plaque by oral bacteria due to the poor oral hygiene. The dental plaque is a mass of bacteria proliferated on the surface of the tooth after adhering thereon using a sticky material included in saliva as an adhesive. If the dental plaque is left untreated, inflammation is developed to cause bleeding in the gingiva and bad breath, which is called gingivitis.

Once gingivitis progresses, the gap between the tooth and the gingiva becomes deeper, and as a result periodontal pocket is formed, wherein bacteria causing periodontal disease are proliferated to cause periodontitis. As periodontitis progresses, a weak stimulus like tooth-brushing may cause bleeding in the gingiva and swelling the same, acute inflammation and pain. Such inflammation decreases the bone forming function but increases the bone absorbing function, so that the alveolar bone is gradually lowered, causing the alveolar bone to be destroyed and eventually to lose teeth. The progressed periodontitis can cause tooth loss. In adults over 40 years of age, periodontitis takes 40% of the causes of teeth removal, indicating that the tooth loss by periodontitis is a serious problem.

The destruction of periodontal ligament and alveolar bone in periodontal disease is due to the degradation of collagen, the main component of those tissues. Although the precise mechanism of such destruction has not been disclosed yet, the degradation of collagen is known to be caused by the collagenase secreted in a host and the neutral protease secreted by periodontal disease-causing bacteria. Therefore, studies to inhibit the progression of periodontal disease by suppressing the activities of collagenase and protease have been made.

For the treatment of periodontal disease, improvement of patients' oral hygiene, non-surgical or surgical dental scaling, dental root planing, gingival curettage, and periodontal regeneration using new attachment have been attempted. However, such surgical treatment methods cause inconvenience of visiting a dental clinic and the efficient treatment is not guaranteed and limited to the progressed disease only, suggesting that there is no preventive effect with this treatment. If this disease is not treated, it progresses to a chronic disease in most cases. Systemic antibiotics and sustained-release formulations have been used as a supplementary treatment. However, there is a serious problem in that drugs are delivered too much to unnecessary areas, resulting in side effects. Recently, periodontopathic bacteria displaying resistance against antibiotics have been reported, making matters worse. When teeth are lost by periodontal disease, prosthodontic treatment or dental implant treatment is required, increasing the burden of patients. In particular, it can be a higher medical barrier for socially and economically vulnerable people. Therefore, if the damaged tissue can be regenerated before the tooth loss, the economic and social burden can be alleviated by reducing the demand for treatments such as artificial teeth or prostheses resulting from the tooth loss.

To overcome the limitations of surgical treatment and the problems of using antibiotics so as to increase the effects of the prevention and treatment of periodontal disease, it is urgently required to develop a new drug that is efficient in recovering the damaged and lost periodontal tissue. The periodontal tissues have all different structural characteristics so that it is hard to establish an efficient treatment method to regenerate the lesion damaged or destroyed by periodontal disease. Periodontal disease can be improved to healing phase by non-surgical or surgical removal of inflammatory connective tissue, such as plaque and calculus. However, the destroyed periodontal tissue cannot be completely recovered by such traditional treatment methods since each of components forming the periodontal tissue cannot be fully recovered.

*Magnolia flower* is the name of the flower bud of magnolia belonging to Magnoliaceae, which tastes a little spicy. In Korea, the dried flower bud of Magnolia denudata Desrousseaux or the congeneric plants thereof is used. In Japan, M. denudata Desr, M. flower Pamp, M. kobus DC, M. salicifolia Maxim, and M. sprengeri Pamp are used. In China, M. denudata Desr, M. flower Pamp, and M. sprengeri Pamp are used. In the oriental medicine, it is known that *Magnolia flower* has a warm character and is efficient in treating headache, low back pain and rhinitis as well as sinusitis. It also has an activity of killing pain, according to the previous report.

US Patent Application No. US 13/739,340 describes a pharmaceutical composition accelerating osteogenesis, which is originated from the extracts of leaves or barks of those plants belonging to Magnoliaceae since they have been known not to have side effects but to be able to promote bone growth and to increase bone density. The human bone maintains its homeostasis through bone modeling and bone remodeling by the action of osteoblasts and osteoclasts. In the early stage of osteogenesis, hyaline cartilage is formed in the form of bone and thereafter calcium is deposited, during which cartilage disappears and bone is growing and cartilage cells turn into osteoblasts.

A tooth is formed by a different process from the above bone formation process. Particularly, when the cells begin to develop at 6 weeks of birth, the surface epithelium begins to invade and the cells begin to be proliferated in the invaginated connective tissue. As the invaginated cells grow in the epithelium, the periodontal tissue is formed below. The cells on the epithelium are differentiated into ameloblasts to cover the tooth, while odontoblasts forming dentine and various dental pulp cells are differentiated in the inside. As described above, the mechanisms and the cells involved in bone formation and tooth formation are completely different from each other.

In the course of study to search a material that can prevent the loss of periodontal tissue caused by periodontitis and accelerate the regeneration of periodontal tissue, the present inventors confirmed that fargesin isolated from the *Magnolia flower* extract could promote the growth of dental root and induce the alveolar bone formation around the teeth efficiently, leading to the completion of the present invention.

### SUMMARY OF THE INVENTION

It is an object of the present invention to provide a pharmaceutical composition for accelerating the growth of dental root or periodontal tissue, or preventing or treating periodontitis, which comprises a *Magnolia flower* extract, a fraction thereof or a compound isolated therefrom as an active ingredient.

To achieve the above object, the present invention provides a pharmaceutical composition for accelerating the growth of dental root or periodontal tissue, or preventing or treating periodontitis, which comprises a *Magnolia flower* extract or a fraction isolated from the same as an active ingredient.

The present invention also provides a quasi-drug composition for accelerating the growth of dental root or periodontal tissue, or preventing or improving periodontitis, which comprises a *Magnolia flower* extract or a fraction isolated from the same as an active ingredient.

The present invention also provides a health functional food for accelerating the growth of dental root or periodontal tissue, or preventing or improving periodontitis, which comprises a *Magnolia flower* extract or a fraction isolated from the same as an active ingredient.

The present invention also provides a cosmetic composition for accelerating the growth of dental root or periodontal tissue, or improving periodontitis, which comprises a *Magnolia flower* extract or a fraction isolated from the same as an active ingredient.

The present invention further provides a pharmaceutical composition for accelerating the growth of dental root or periodontal tissue, or preventing or treating periodontitis, which comprises the compound represented by formula 1 or formula 2 below, the pharmaceutically acceptable salt thereof, or the optical isomer thereof as an active ingredient: 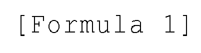 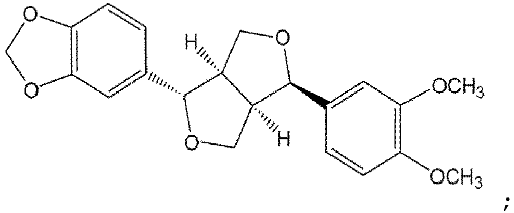 and

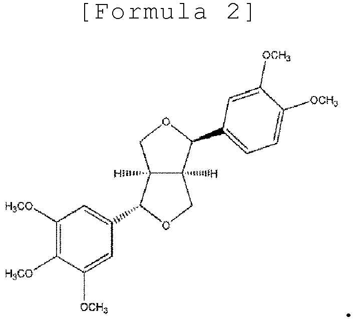

The present invention also provides a quasi-drug composition for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient.

The present invention also provides a health functional food for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient.

The present invention also provides a cosmetic composition for accelerating the growth of dental root or periodontal tissue, or improving periodontitis, which comprises the compound represented by formula 1 or formula 2, the pharmaceutically acceptable salt thereof, or the optical isomer thereof as an active ingredient.

The present invention also provides a method for isolating the compound represented by formula 1 or formula 2 above comprising the following steps:
1) producing a methanol fraction by adding methanol to the ethyl acetate layer generated by the fractionation of the *Magnolia flower* extract with ethyl acetate;
2) obtaining an eluate by adding the mixed solvent comprising ethyl acetate and hexane to the methanol fraction of step 1); and
3) precipitating crystals in the eluate of step 2), followed by filtering thereof with a filter.

The present invention also provides a method for accelerating the growth of dental root or periodontal tissue, or preventing or treating periodontitis comprising the step of administering a pharmaceutically effective dose of a *Magnolia flower* extract or a fraction isolated from the same to a subject.

The present invention also provides a use of the pharmaceutical composition comprising a *Magnolia flower* extract or a fraction isolated therefrom as an active ingredient for accelerating the growth of dental root or periodontal tissue, or preventing or treating periodontitis.

The present invention also provides a use of the quasi-drug composition comprising a *Magnolia flower* extract or a fraction isolated therefrom as an active ingredient for accelerating the growth of dental root or periodontal tissue, or preventing or improving periodontitis.

The present invention also provides a use of the health functional food comprising a *Magnolia flower* extract or a fraction isolated therefrom as an active ingredient for accelerating the growth of dental root or periodontal tissue, or preventing or improving periodontitis.

The present invention also provides a use of the cosmetic composition comprising a *Magnolia flower* extract or a fraction isolated therefrom as an active ingredient for accelerating the growth of dental root or periodontal tissue, or improving periodontitis.

The present invention also provides a method for accelerating the growth of dental root or periodontal tissue, or preventing or treating periodontitis comprising the step of administering a pharmaceutically effective dose of the compound represented by formula 1 or formula 2, the pharmaceutically acceptable salt thereof, or the optical isomer thereof to a subject.

The present invention also provides a use of the pharmaceutical composition comprising the compound represented by formula 1 or formula 2, the pharmaceutically acceptable salt thereof, or the optical isomer thereof as an active ingredient for accelerating the growth of dental root or periodontal tissue, or preventing or treating periodontitis.

The present invention also provides a use of the quasi-drug composition comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

The present invention also provides a use of the health functional food comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

The present invention also provides a use of the cosmetic composition comprising the compound represented by formula 1 or formula 2, the pharmaceutically acceptable salt thereof, or the optical isomer thereof as an active ingredient for accelerating the growth of dental root or periodontal tissue, or improving periodontitis.

### ADVANTAGEOUS EFFECT

The *Magnolia flower* extract, the fraction thereof, and the compound represented by formula 1 or formula 2 below of the present invention can promote the growth of dental root; promote the growth of periodontal tissue by forming alveolar bone around the teeth; and inhibit periodontitis, so that the *Magnolia flower* extract, the fraction thereof, and the compound of the invention can be effectively used as an active ingredient of a pharmaceutical composition for accelerating the growth of dental root or periodontal tissue, or preventing or treating periodontitis:

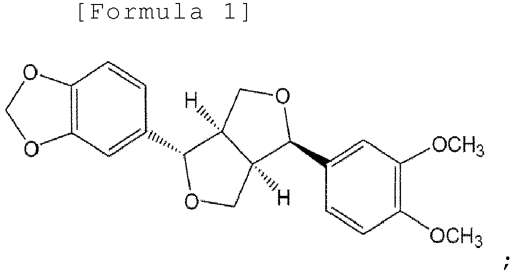

and

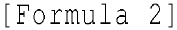

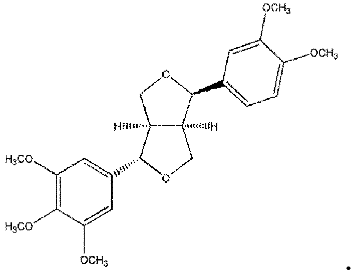

### BRIEF DESCRIPTION OF THE DRAWINGS

The application of the preferred embodiments of the present invention is best understood with reference to the accompanying drawings, wherein:
Figure 1 is a diagram illustrating the structure of human teeth.

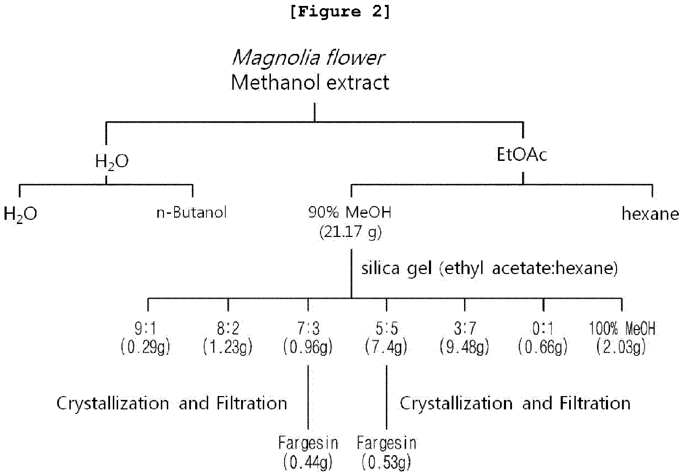

Figure 2 is a diagram illustrating the process for producing the *Magnolia flower* extract, the fraction thereof, and the compound above.
Figure 3 is a diagram illustrating the results of the transplantation of the tooth embedded in agarose beads containing fargesin to a host mouse:
   PBS: a tooth embedded in agarose beads containing PBS; and
   Fargesin: a tooth embedded in agarose beads containing fargesin.
Figure 4 is a diagram illustrating the results of the transplantation of the tooth embedded in agarose beads containing the *Magnolia flower* extract:
   PBS: a tooth embedded in agarose beads containing PBS;
   Fargesin: a tooth embedded in agarose beads containing fargesin;
   Low Conc.: a tooth embedded in agarose beads containing a low concentration of the *Magnolia flower* extract; and
   High Conc.: a tooth embedded in agarose beads containing a high concentration of the *Magnolia flower* extract.

### DESCRIPTION OF THE PREFERRED EMBODIMENTS

Hereinafter, the present invention is described in detail.

The present invention provides a pharmaceutical composition for accelerating the growth of dental root or periodontal tissue, or preventing or treating periodontitis, which comprises a *Magnolia flower* extract or a fraction isolated from the same as an active ingredient.

The present invention also provides a method for accelerating the growth of dental root or periodontal tissue, or preventing or treating periodontitis comprising the step of administering a pharmaceutically effective dose of a *Magnolia flower* extract or a fraction isolated from the same to a subject.

The present invention also provides a use of the pharmaceutical composition comprising a *Magnolia flower* extract or a fraction isolated therefrom as an active ingredient for accelerating the growth of dental root or periodontal tissue, or preventing or treating periodontitis.

The *Magnolia flower* can be purchased or cultivated. All of the parts of *Magnolia flower* such as stems, leaves, roots, and flower buds, etc, can be used, but in a preferred embodiment of the present invention, flower buds were used.

The method for the extraction of *Magnolia flower* can be one of the conventional methods accepted in this field, which is exemplified by filtration, hot-water extraction, enfleurage, reflux extraction, and ultrasonification extraction. When the extraction is performed by hot-water extraction, the extraction times are preferably 1 ∼ 5 times and more preferably 3 times, but not always limited thereto. The extraction solvent can be added to the dried *Magnolia flower* at the volume of 0.1 ∼ 10 times the volume of the dried *Magnolia flower,* and more preferably added by 0.3 ∼ 5 times the volume of the dried *Magnolia flower.* The extraction temperature is preferably 20°C ∼ 40°C and the extraction time is preferably 12 ∼ 48 hours.

The *Magnolia flower* extract or the fraction thereof can be prepared by the preparation method comprising the following steps:
1) drying *Magnolia flower* to remove moisture and then pulverizing the dried *Magnolia flower;*
2) extracting the dried *Magnolia flower* with an extraction solvent at room temperature;
3) filtering the extract obtained in step 2, followed by concentration under reduced pressure; and
4) fractionating the extract by adding an organic solvent.

In the method above, drying in step 1) is preferably performed by reduced-pressurized drying, vacuum drying, boiling drying, spray drying, or freeze drying, but not always limited thereto.

In the method above, the extraction solvent of step 2) can be water, C₁ ∼ C₂ lower alcohol, or a mixed solvent thereof.

In this method, concentration under reduced pressure in step 3) is preferably performed by using a vacuum concentrator or a vacuum rotary evaporator, but not always limited thereto.

In the method above, the organic solvent of step 4) can be ethyl acetate, hexane, benzene, or chloroform.

The said fraction can be prepared by the following process: fractionating the *Magnolia flower* extract by using ethyl acetate and then performing additional fractionation with the fraction by using methanol; or performing additional fractionation with the fraction by using a mixed solvent comprising ethyl acetate and hexane at the ratio of 3:7 or 5:5.

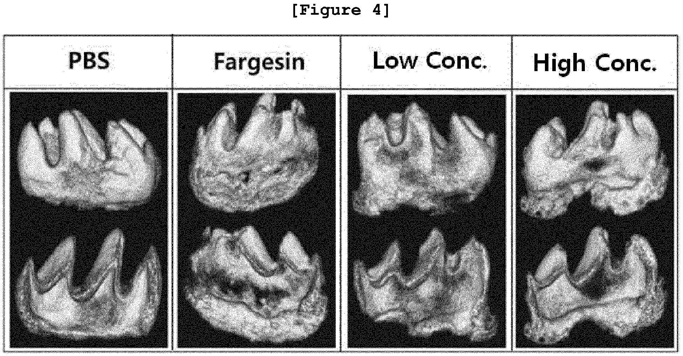

In a preferred embodiment of the present invention, the present inventors confirmed that when the tooth was embedded in agarose beads containing the *Magnolia flower* extract and then cultured, the growth of dental root was significantly promoted and the alveolar bone was newly formed around the tooth (see Figure 4), indicating that the *Magnolia flower* extract was efficient in promoting the growth of dental root or periodontal tissue.

Regarding the results of the above-mentioned embodiment, when a tooth was extracted from the mouse at the age of 5 days, it was extracted not only the tooth but also the periodontal ligament which can help the growth of the tooth and dental root as being attached to the tooth because the tooth was taken out as it was in the inside of the alveolar bone. The dental root and periodontal tissue were grown by the sample in the extracted tooth. At that time, various cells included in the periodontal ligament were interacted to help the growth of dental root. The outer surface of the dental root is covered with the hard tissue called cementum, which is connected to the periodontal ligament and to fix teeth. The cementum is also formed by periodontal ligament cells. So, the interaction among various cells included in the periodontal ligament not only induces the growth of dental root but also forms the cementum surrounding the dental root. Thus, the periodontal ligament is an important factor for the growth of dental root and periodontal tissue.

Accordingly, the *Magnolia flower* extract or the fraction thereof can be effectively used as a pharmaceutical composition for promoting the growth of dental root or periodontal tissue, or preventing or treating periodontitis.

The composition of the present invention preferably includes the *Magnolia flower* extract of the invention by 0.1 ∼ 99.9 weight% for the total weight of the composition, but not always limited thereto. The composition can additionally include pharmaceutically acceptable carriers, excipients or diluents.

The composition of the present invention can be administered orally or parenterally and be used in general forms of pharmaceutical formulation. That is, the composition of the present invention can be prepared for oral or parenteral administration by mixing with generally used diluents or excipients such as fillers, extenders, binders, wetting agents, disintegrating agents and surfactants. Solid formulations for oral administration are tablets, pills, powders, granules and capsules. These solid formulations are prepared by mixing the compound with one or more suitable excipients such as starch, calcium carbonate, sucrose or lactose, gelatin, etc. Except for the simple excipients, lubricants, for example magnesium stearate, talc, etc, can be used. Liquid formulations for oral administrations are suspensions, solutions, emulsions and syrups, and the above-mentioned formulations can contain various excipients such as wetting agents, sweeteners, aromatics and preservatives in addition to generally used simple diluents such as water and liquid paraffin. Formulations for parenteral administration are sterilized aqueous solutions, water-insoluble excipients, suspensions, emulsions, lyophilized preparations and suppositories.

Water insoluble excipients and suspensions can contain, in addition to the active compound or compounds, propylene glycol, polyethylene glycol, vegetable oil like olive oil, injectable ester like ethylolate, etc. Suppositories can contain, in addition to the active compound or compounds, witepsol, macrogol, tween 61, cacao butter, laurin butter, glycerogelatin, etc.

The composition of the present invention can be administered orally or parenterally. The parenteral administration herein can be performed by external skin application, intraperitoneal injection, intrarectal injection, intravenous injection, intramuscular injection, hypodermic injection, intrauterine injection, or intracerebroventricular injection. Among them external skin application is most preferred.

The effective dosage of the composition of the present invention can be determined according to weight, age, gender, health condition, diet, administration frequency, administration method, excretion and severity of a disease. The dosage is 0.01 ∼ 1000 mg/kg per day, preferably 30 ∼ 500 mg/kg per day, and more preferably 50 ∼ 300 mg/kg per day, and administration frequency is preferably 1 ∼ 6 times a day.

The composition of the present invention can be administered alone or treated together with surgical operation, radio-therapy, hormone therapy, chemotherapy and biological regulators.

The present invention also provides a quasi-drug composition for accelerating the growth of dental root or periodontal tissue, or preventing or improving periodontitis, which comprises a *Magnolia flower* extract or a fraction isolated from the same as an active ingredient.

The present invention also provides a use of the quasi-drug composition comprising a *Magnolia flower* extract or a fraction isolated therefrom as an active ingredient for accelerating the growth of dental root or periodontal tissue, or preventing or improving periodontitis.

The quasi-drug composition is preferably an oral composition formulation such as toothpaste, mouthwash, or mouthcleaner.

The quasi-drug composition comprising the *Magnolia flower* extract or the fraction thereof of the present invention is not limited in the formulation and can be formulated in the conventional form such as toothpaste, mouthwash, or mouthcleaner. The oral composition provided in the present invention can contain various bases and additives necessary for the formulation, and the kind and amount of these ingredients can be easily selected by those in the art. For example, when the oral composition is formulated as toothpaste, it can include an abrasive, a wetting agent, a foaming agent, a binder, a sweetener, a pH regulator, a preservative, a medicinal ingredient, a flavoring agent, a brightener, a colorant, and a solvent.

The present invention also provides a health functional food for accelerating the growth of dental root or periodontal tissue, or preventing or improving periodontitis, which comprises a *Magnolia flower* extract or a fraction isolated from the same as an active ingredient.

The present invention also provides a use of the health functional food comprising a *Magnolia flower* extract or a fraction isolated therefrom as an active ingredient for accelerating the growth of dental root or periodontal tissue, or preventing or improving periodontitis.

The health functional food of the present invention can additionally include various flavors or natural carbohydrates. The natural carbohydrates above can be one of monosaccharides such as glucose and fructose, disaccharides such as maltose and sucrose, polysaccharides such as dextrin and cyclodextrin, and glucose alcohols such as xilytole, sorbitol and erythritol. Besides, natural sweetening agents such as thaumatin and stevia extract, and synthetic sweetening agents such as saccharin and aspartame can be included as a sweetening agent. The content of the natural carbohydrate is preferably 0.01 ∼ 0.04 weight part and more preferably 0.02 ∼ 0.03 weight part in 100 weight part of the health functional food of the present invention.

In addition to the ingredients mentioned above, the health functional food of the present invention can include in a variety of nutrients, vitamins, minerals, flavors, coloring agents, pectic acid and its salts, alginic acid and its salts, organic acid, protective colloidal viscosifiers, pH regulators, stabilizers, antiseptics, glycerin, alcohols, carbonators which used to be added to soda, etc. All the mentioned ingredients can be added singly or together. The mixing ratio of those ingredients does not matter in fact, but in general, each can be added by 0.01 ∼ 0.1 weight part per 100 weight part of the health functional food of the present invention.

The food herein is not limited. For example, the extract of the present invention can be added to meats, sausages, breads, chocolates, candies, snacks, cookies, pizza, ramyuns, flour products, gums, dairy products including ice cream, soups, beverages, tea, drinks, alcohol drinks and vitamin complex, etc, and in wide sense, almost every food applicable in the production of health food can be included.

The present invention also provides a cosmetic composition for accelerating the growth of dental root or periodontal tissue, or improving periodontitis, which comprises a *Magnolia flower* extract or a fraction isolated from the same as an active ingredient.

The present invention also provides a use of the cosmetic composition comprising a *Magnolia flower* extract or a fraction isolated therefrom as an active ingredient for accelerating the growth of dental root or periodontal tissue, or improving periodontitis.

The cosmetic composition of the present invention can be formulated in any form that can be accepted in the art, which is exemplified by lotions, ointments, gels, creams, patches or sprays. In preparing the cosmetic composition comprising the *Magnolia flower* extract or the fraction thereof of the present invention, the *Magnolia flower* extract or the fraction thereof can be added by 3 ∼ 30 weight part, preferably 5 ∼ 20 weight part to the cosmetic composition of the present invention.

The cosmetic composition of the present invention can additionally include a supplement generally used in the field of skin science such as fatty substance, organic solvent, resolvent, concentrate, gelling agent, softener, antioxidant, suspending agent, stabilizer, foaming agent, odorant, surfactant, water, ionic or non-ionic emulsifying agent, filler, sequestering agent, chelating agent, preserving agent, vitamin, blocker, moisturing agent, essential oil, dye, pigment, hydrophilic or hydrophobic activator, lipid vesicle or other components generally used in a cosmetic composition.

The amount of the above supplement can be determined as generally accepted in the field of skin science.

The present invention also provides a pharmaceutical composition for accelerating the growth of dental root or periodontal tissue, or preventing or treating periodontitis, which comprises the compound represented by formula 1 or formula 2 below, the pharmaceutically acceptable salt thereof, or the optical isomer thereof as an active ingredient

The present invention also provides a method for accelerating the growth of dental root or periodontal tissue, or preventing or treating periodontitis comprising the step of administering a pharmaceutically effective dose of the compound represented by formula 1 or formula 2 below, the pharmaceutically acceptable salt thereof, or the optical isomer thereof to a subject.

The present invention also provides a use of the pharmaceutical composition comprising the compound represented by formula 1 or formula 2 below, the pharmaceutically acceptable salt thereof, or the optical isomer thereof as an active ingredient for accelerating the growth of dental root or periodontal tissue, or preventing or treating periodontitis.

The compound represented by formula 1 or formula 2 below can be isolated from the *Magnolia flower* extract.

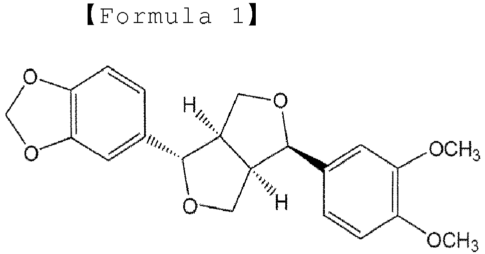

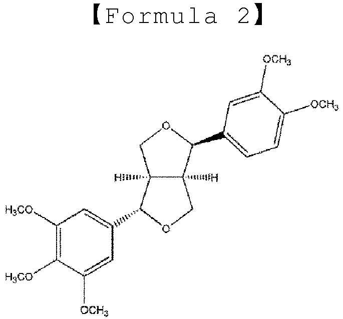

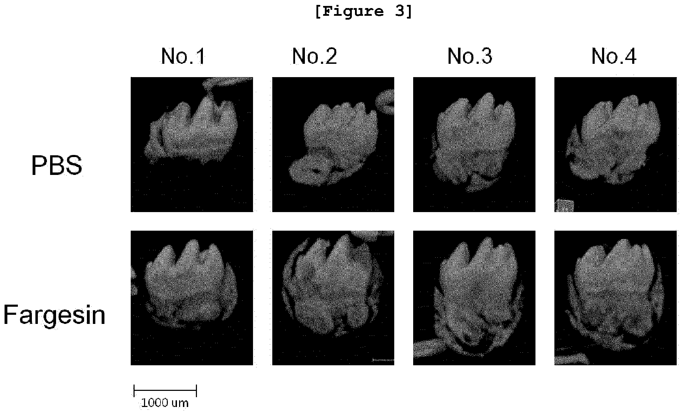

In a preferred embodiment of the present invention, the present inventors confirmed that when the tooth was embedded in agarose beads containing the fargesin isolated from the *Magnolia flower* extract and then cultured, the growth of dental root was significantly promoted and the alveolar bone was newly formed around the tooth (see Figure 3), indicating that the compound represented by formula 1 or formula 2 was efficient in promoting the growth of dental root or periodontal tissue, or preventing or treating periodontitis.

The compound represented by formula 1 or formula 2 of the present invention can be used as the form of a pharmaceutically acceptable salt, in which the salt is preferably acid addition salt formed by pharmaceutically acceptable free acids. The acid addition salt can be obtained from inorganic acids such as hydrochloric acid, nitric acid, phosphoric acid, sulfuric acid, hydrobromic acid, hydroiodic acid, nitrous acid and phosphorous acid, or non-toxic organic acids such as aliphatic mono/dicarboxylate, phenyl-substituted alkanoate, hydroxy alkanoate, alkandioate, aromatic acids and aliphatic/aromatic sulfonic acids. The pharmaceutically non-toxic salts are exemplified by sulfate, pyrosulfate, bisulfate, sulphite, bisulphite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutylate, caprate, heptanoate, propiolate, oxalate, malonate, succinate, suberate, cabacate, fumarate, maliate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutylate, citrate, lactate, hydroxybutylate, glycolate, malate, tartrate, methanesulfonate, propanesulfonate, naphthalene-1-sulfonate, naphthalene-2-sulfonate and mandelate.

The acid addition salt in this invention can be prepared by the conventional method known to those in the art. For example, the compound represented by formula 1 or formula 2 of the present invention is dissolved in an organic solvent such as methanol, ethanol, acetone, methylenechloride, or acetonitrile, to which organic acid or inorganic acid is added to induce precipitation. Then, the precipitate is filtered and dried to give the salt. Or the solvent and the excessive acid are distillated under reduced pressure, and dried to give the salt. Or the precipitate is crystallized in the organic solvent to give the same.

A pharmaceutically acceptable metal salt can be prepared by using a base. Alkali metal or alkali earth metal salt is obtained by the following processes: dissolving the compound in excessive alkali metal hydroxide or alkali earth metal hydroxide solution; filtering non-soluble compound salt; evaporating the remaining solution and drying thereof. At this time, the metal salt is preferably prepared in the pharmaceutically suitable form of sodium, potassium, or calcium salt. And the corresponding silver salt is prepared by the reaction of alkali metal or alkali earth metal salt with proper silver salt (ex; silver nitrate).

The present invention includes not only the compound represented by formula 1 or formula 2 and the pharmaceutically acceptable salt thereof, but also solvates, hydrates, and isomers possibly produced from the same.

The present invention also provides a quasi-drug composition for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient

The present invention also provides a use of the quasi-drug composition comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

The present invention also provides a health functional food for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient

The present invention also provides a use of the health functional food comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

The present invention also provides a cosmetic composition for accelerating the growth of dental root or periodontal tissue, or improving periodontitis, which comprises the compound represented by formula 1 or formula 2, the pharmaceutically acceptable salt thereof, or the optical isomer thereof as an active ingredient

The present invention also provides a use of the cosmetic composition comprising the compound represented by formula 1 or formula 2, the pharmaceutically acceptable salt thereof, or the optical isomer thereof as an active ingredient for accelerating the growth of dental root or periodontal tissue, or improving periodontitis.

In a preferred embodiment of the present invention, the present inventors confirmed that when the tooth was embedded in agarose beads containing the fargesin isolated from the *Magnolia flower* extract and then cultured, the growth of dental root was significantly promoted and the alveolar bone was newly formed around the tooth (see Figure 3), indicating that the compound represented by formula 1 or formula 2 can be effectively used as an active ingredient of a quasi-drug composition, a cosmetic composition or a health functional food for promoting the growth of dental root or periodontal tissue, or preventing or improving periodontitis.

In addition, the present invention provides a method for isolating the compound represented by formula 1 or formula 2 above comprising the following steps:
1) producing a methanol fraction by adding methanol to the ethyl acetate layer generated by the fractionation of the *Magnolia flower* extract with ethyl acetate;
2) obtaining an eluate by adding the mixed solvent comprising ethyl acetate and hexane to the methanol fraction of step 1); and
3) precipitating crystals in the eluate of step 2), followed by filtering thereof with a filter.

The *Magnolia flower* extract above is preferably extracted by using water, C₁ ∼ C₂ lower alcohol, or a mixed solvent thereof. The mixed solvent of ethyl acetate and hexane of step 2 comprises ethyl acetate and hexane at the ratio of 3:7 or 5:5.

Practical and presently preferred embodiments of the present invention are illustrative as shown in the following Examples.

However, it will be appreciated that those skilled in the art, on consideration of this disclosure, may make modifications and improvements within the spirit and scope of the present invention.

### Example 1: Preparation of Magnolia flower extract, fraction, and compound

The *Magnolia flower* extract, the fraction, and the compound used in this invention were prepared by the following method.

### <1-1> Preparation of Magnolia flower extract

Methanol was added to Chinese *Magnolia flower* (purchased from 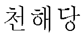 , Osaka, Japan) at the volume of 10 times the volume of the flower bud, followed by extraction for 3 ∼ 7 days. The extract was filtered to remove insoluble residue, and the filtered extract was concentrated using a vacuum concentrator. Then, the solid *Magnolia flower* extract was obtained by freeze-drying the concentrated extract.

### <1-2> Preparation of Magnolia flower fraction

The extract obtained in Example <1-1> was fractionated with ethyl acetate and distilled water. Then, the distilled water layer was fractionated with butanol, and the ethyl acetate layer was further fractionated with 90% methanol and hexane. The 90% methanol layer (21.17 g) was fractionated into seven eluates (1:9, 2:8, 3:7, 5:5, 100% ethyl acetate, and 100% methanol) using silica gel and a mixed solvent of hexane and ethyl acetate (ethyl acetate:hexane).

### <1-3> Compound isolation

Among the 7 eluates obtained in Example <1-2> above, crystals were precipitated in two elates (ethyl acetate:hexane = 3:7 and 5:5). The crystals were filtered with a filter and as a result, 0.44 g and 0.53 g of fargesin were obtained.

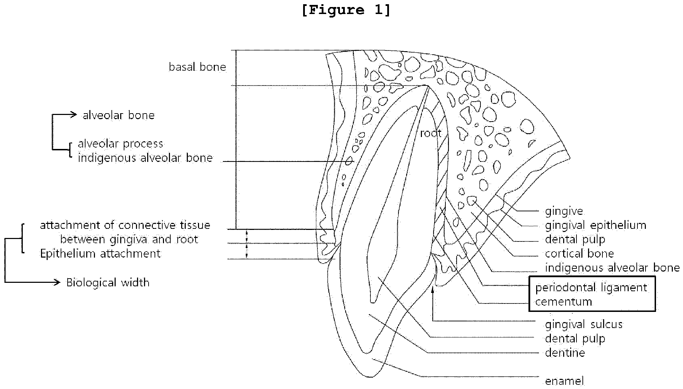

The preparation process of the *Magnolia flower* extract, the fraction, and the compound is illustrated in Figure 1 (Figure 1).

### Example 2: Solvent fractionation for increasing the active ingredient content in the Magnolia flower extract

To increase the contents of fargesin and magnolin, the lignan compounds, in the *Magnolia flower* extract, fractions were obtained with different conditions as shown below and the contents of fargesin and magnolin were measured.

### <2-1> Solvent fractionation by pH

1.2 ℓ of 70% ethanol (v/v) was added to 100 g of the chopped *Magnolia flower,* followed by reflux extraction at 85□ for 6 hours. Then, the extract was filtered, and the residue was washed with 70% ethanol. The filtrate was collected and concentrated under reduced pressure. As a result, 17 g of 70% ethanol extract was obtained. Water was added to the extract obtained above and completely dissolved. Then, pH was adjusted according to the conditions (pH 5.0, pH 3.0, and pH 9.0). N-Hexane was added to water, followed by solvent fractionation several times. Concentration was performed under reduced pressure. The contents of fargesin and magnolin were analyzed by fargesin content test and fingerprint pattern assay.

As a result, as shown in Table 1, fargesin and magnolin were detected in both hexane and water fractions in the control group wherein pH was not regulated. At that time, the recovery rate of water fraction was higher than that of hexane fraction (Table 1).

**[Table 1]**

| Compound | Solvent | Form | Yield (%) | Content (%) | Content (mg) | Recovery rate (%) |
|---|---|---|---|---|---|---|
| Fargesin | 70% ethanol | gum | 26.7 | 1.1 | 36 | NA |
| | n-hexane | oil | 5.1 | 2.1 | 13 | 36 |
| | water | powder | 25.8 | 0.7 | 22 | 61 |
| Magnolin | 70% ethanol | gum | 26.7 | 15.2 | 580 | NA |
| | n-hexane | oil | 5.1 | 14.2 | 87 | 15 |
| | water | powder | 25.8 | 15.8 | 490 | 84 |

As shown in Table 2, when fractionation was performed in the acidic condition (pH 3.0), the overall recovery rate was decreased because the furofuran ring of lignan was opened and degraded.

**[Table 2]**

| Compound | Solvent | Form | Yield (%) | Content (%) | Content (mg) | Recovery rate (%) |
|---|---|---|---|---|---|---|
| Fargesin | 70% ethanol | gum | 26.7 | 1.1 | 36 | NA |
| | n-hexane | oil | 5.1 | 2.0 | 12 | 33 |
| | water | powder | 25.8 | 0.24 | 8 | 22 |
| Magnolin | 70% ethanol | gum | 26.7 | 15.2 | 580 | NA |
| | n-hexane | oil | 5.1 | 12.8 | 77 | 13 |
| | water | powder | 25.8 | 5.3 | 163 | 28 |

As shown in table 3, when fractionation was performed in the basic condition (pH 9.0), the overall recovery rate was increased, compared with that in the acidic condition, and the relative content was increased due to other materials reacting to the basic condition.

**[Table 3]**

| Compound | Solvent | Form | Yield (% ) | Content (% ) | Content (mg) | Recovery rate (%) |
|---|---|---|---|---|---|---|
| Fargesin | 70% ethanol | gum | 26.7 | 1.1 | 36 | NA |
| | n-hexane | oil | 5.1 | 3.1 | 15 | 42 |
| | water | powder | 25.8 | 0.6 | 20 | 55 |
| Magnolin | 70% ethanol | gum | 26.7 | 15.2 | 580 | NA |
| | n-hexane | oil | 5.1 | 19.8 | 96 | 17 |
| | water | powder | 25.8 | 14.8 | 465 | 80 |

### Experimental Example 1: Growth promoting effect of fargesin isolated from Magnolia flower extract on dental root or periodontal tissue

The following experiment was performed in order to investigate the promoting effect of the fargesin isolated from the *Magnolia flower* extract on the growth of dental root or periodontal tissue.

Particularly, the fargesin obtained in Example 1 was diluted in PBS (Dainippon Pharmaceuticals Inc., Japan) at the final concentration of 80 *µ*m, and four agarose beads (Affi-Gel Blue Gel, #153-7301, Bio-Rad Laboratories Inc., Japan) were washed with PBS, followed by reaction for at least 60 minutes to allow the fargesin to be absorbed in those beads. For the control, 4 other beads were reacted only with PBS. A 5-day-old C57BL/6 mouse (Sankyo Labo Service Corporation, Japan) was sacrificed, from which molars were extracted from the lower jaws. Dental pulp of the extracted molar was embedded in the agarose beads containing fargesin. For the control, dental pulp of the extracted molar was embedded in the agarose beads containing PBS alone. The agarose beads were each grafted into capsules of the 8-week-old C57BL/6 host mouse (female, Sankyo Labo Service Corporation, Japan) kidneys. The host mice were raised in the conditions of 12h/12h light and dark cycle, room temperature, and free feeding. 3 weeks later, the mice were sacrificed, from which the kidneys were extracted. Then, the teeth were observed. The 3D image of the teeth was taken by micro CT (Rigaku R_mCT2).

As a result, as shown in Figure 3, the growth of dental root and bone was hardly observed in the PBS control group, while the growth of dental root was significant and the formation of alveolar bone around the grafted tooth was observed in the fargesin experimental group (Figure 3). Therefore, it was confirmed that the fargesin compound isolated from the *Magnolia flower* extract of the present invention was efficient in promoting the growth of dental root or the formation of periodontal tissue.

### Experimental Example 2: Growth promoting effect of Magnolia flower extract on dental root and periodontal tissue

The following experiment was performed in order to investigate the promoting effect of the *Magnolia flower* extract on the growth of dental root and periodontal tissue.

Particularly, the *Magnolia flower* extract obtained in Example 1 was diluted in PBS at the final concentrations of 50 and 500 ug/ml. Four agarose beads (Affi-Gel Blue Gel, #153-7301, Bio-Rad Laboratories Inc., Korea) were washed with PBS, followed by reaction for at least 60 minutes to allow the fargesin to be absorbed in those beads. For the control, 4 other beads were reacted only with PBS. For the comparative group, beads were reacted with the fargesin compound as shown in Experimental Example 1. A 5-day-old C57BL/6 mouse (DBL Co., Ltd., Korea) was sacrificed, from which molars were extracted from the lower jaws. Dental pulp of the extracted molar was embedded in the agarose beads containing fargesin or the *Magnolia flower* extract. For the control, dental pulp of the extracted molar was embedded in the agarose beads containing PBS alone. The agarose beads were each grafted into capsules of the 8-week-old C57BL/6 host mouse (female, DBL Co., Ltd., Korea) kidneys. The host mice were raised in the conditions of 12h/12h light and dark cycle, room temperature, and free feeding. 3 weeks later, the mice were sacrificed, from which the kidneys were extracted. Then, the teeth were observed. The 3D image of the teeth was taken by micro CT.

As a result, as shown in Figure 4, the growth of dental root and bone was hardly observed in the PBS control group, while the growth of dental root was significant in the experimental group the *Magnolia flower* extract dose-dependently and the formation of alveolar bone around the grafted tooth was observed (Figure 4). Therefore, it was confirmed that the *Magnolia flower* extract was efficient in promoting the growth of dental root and the formation of periodontal tissue as much as or better than the fargesin compound.

Hereinafter, Manufacturing Examples of a pharmaceutical composition, a quasi-drug composition, and a cosmetic composition comprising the *Magnolia flower* extract of the present invention, the fraction thereof, or the compound isolated from the same as an active ingredient are described.

### Manufacturing Example 1: Preparation of pharmaceutical formulation

### <1-1> Preparation of tablets (direct pressurization)

| | |
|---|---|
| Active ingredient | 5.0 mg |
| Lactose | 14.1 mg |
| Crospovidone USNF | 0.8 mg |
| Magnesium stearate | 0.1 mg |

All the above components were added in the prescribed amounts, uniformly mixed and pressed to prepare tablets according to the conventional method for preparing tablets.

### <1-2> Preparation of tablets (slurry granulation)

| | |
|---|---|
| Active ingredient | 5.0 mg |
| Lactose | 16.0 mg |
| Starch | 4.0 mg |
| Polysorbate 80 | 0.3 mg |
| Silicon dioxide | 2.7 mg |
| Magnesium stearate | 2.0 mg |

After sieving active ingredient, lactose and starch were added and mixed. After polysorbate was dissolved in pure water, an appropriate amount of the solution was added to the mixture of active ingredient, lactose and starch, followed by atomization. After drying, the granules were sieved and mixed with colloidal silicon dioxide and magnesium stearate. The granules were pressurized to prepare tablets.

### Manufacturing Example 2: Preparation of quasi-drug composition

### <2-1> Preparation of oral gargle

| | |
|---|---|
| Active ingredient | 0.1 ∼ 1.0 g |
| Xylitol | 5 ∼ 10 g |
| Ethyl alcohol | 5 ∼ 15 g |
| Sorbitol | 5 ∼ 15 g |
| Sodium saccharin | 10 ∼ 100 mg |
| Sodium monofluorophosphate | 500 ∼ 1000 mg |
| Sodium lauryl sulfate | 100 ∼ 200 mg |
| Polysorbate 20 | 100 ∼ 1000 mg |
| Peppermint flavor | 10 ∼ 100 mg |
| Sodium benzoate | 10 ∼ 100 g |

### <2-2> Preparation of toothpaste

| | |
|---|---|
| Active ingredient | 1.9 weight part |
| Silicic anhydride | 10.0 weight part |
| Xanthan gum | 0.7 weight part |
| Magnesium sulfate | 2.0 weight part |
| Sodium monofluorophosphate | 0.7 weight part |
| Sorbitol | 59.0 weight part |
| Polyethylene glycol | 4.0 weight part |
| Sodium saccharin | 0.2 weight part |
| Sodium lauryl sulfate | 1.0 weight part |
| Flavor | 0.5 weight part |
| Purified water | 20.0 weight part |

### <2-3> Preparation of liquid toothpaste

| | |
|---|---|
| Active ingredient | 2.2 weight part |
| Sodium monofluorophosphate | 0.7 weight part |
| Sorbitol | 54.0 weight part |
| Concentrated glycerin | 2.0 weight part |
| Ethanol | 5.0 weight part |
| Magnesium sulfate | 2.0 weight part |
| Sodium saccharin | 0.2 weight part |
| Carrageenan | 0.5 weight part |
| Sodium lauryl sulfate | 0.5 weight part |
| Polyglycerin fatty acid ester | 2.0 weight part |
| Flavor | 0.9 weight part |
| Purified water | 30 weight part |

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended Claims.

## Claims

1. A pharmaceutical composition for accelerating the growth of dental root or periodontal tissue, or preventing or treating periodontitis, which comprises a *Magnolia flower* extract or a fraction isolated from the same as an active ingredient.

2. The pharmaceutical composition for accelerating the growth of dental root or periodontal tissue, or preventing or treating periodontitis according to claim 1, wherein the *Magnolia flower* extract is extracted by using water, C₁ ∼ C₂ lower alcohol, or a mixed solvent thereof as an extraction solvent.

3. The pharmaceutical composition for accelerating the growth of dental root or periodontal tissue, or preventing or treating periodontitis according to claim 1, wherein the fraction is obtained by fractionating the *Magnolia flower* extract with ethyl acetate, hexane, benzene, or chloroform.

4. The pharmaceutical composition for accelerating the growth of dental root or periodontal tissue, or preventing or treating periodontitis according to claim 1, wherein the fraction is obtained by fractionating the *Magnolia flower* extract with ethyl acetate and then performing additional fractionation with the fraction by using methanol; or performing additional fractionation with the fraction by using a mixed solvent comprising ethyl acetate and hexane at the ratio of 3:7 or 5:5.

5. A quasi-drug composition for accelerating the growth of dental root or periodontal tissue, or preventing or improving periodontitis, which comprises a *Magnolia flower* extract or a fraction isolated from the same as an active ingredient.

6. The quasi-drug composition for accelerating the growth of dental root or periodontal tissue, or preventing or improving periodontitis according to claim 5, wherein the quasi-drug composition is in the form of toothpaste, mouthwash, or mouthcleaner.

7. A health functional food for accelerating the growth of dental root or periodontal tissue, or preventing or improving periodontitis, which comprises a *Magnolia flower* extract or a fraction isolated from the same as an active ingredient.

8. A cosmetic composition for accelerating the growth of dental root or periodontal tissue, or improving periodontitis, which comprises a *Magnolia flower* extract or a fraction isolated from the same as an active ingredient.

9. A pharmaceutical composition for accelerating the growth of dental root or periodontal tissue, or preventing or treating periodontitis, which comprises the compound represented by formula 1 or formula 2 below, the pharmaceutically acceptable salt thereof, or the optical isomer thereof as an active ingredient: 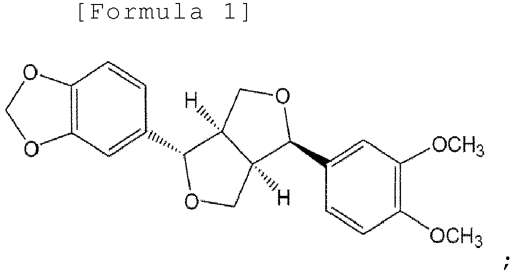 and

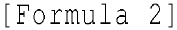

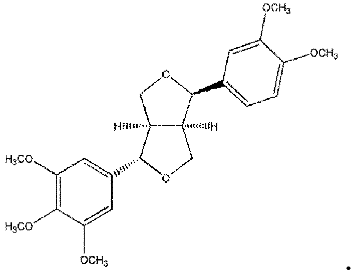

10. The pharmaceutical composition for accelerating the growth of dental root or periodontal tissue, or preventing or treating periodontitis according to claim 9, wherein the compound represented by formula 1 or formula 2 above is isolated from the *Magnolia flower* extract.

11. A quasi-drug composition for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient.

12. The quasi-drug composition for accelerating the growth of dental root or periodontal tissue, or preventing or improving periodontitis according to claim 11, wherein the quasi-drug composition is in the form of toothpaste, mouthwash, or mouthcleaner.

13. A health functional food for accelerating the growth of dental root or periodontal tissue, or preventing or improving periodontitis, which comprises the compound represented by formula 1 or formula 2, the pharmaceutically acceptable salt thereof, or the optical isomer thereof as an active ingredient.

14. A cosmetic composition for accelerating the growth of dental root or periodontal tissue, or improving periodontitis, which comprises the compound represented by formula 1 or formula 2, the pharmaceutically acceptable salt thereof, or the optical isomer thereof as an active ingredient.

15. A method for isolating the compound represented by formula 1 or formula 2 above comprising the following steps:
1) producing a methanol fraction by adding methanol to the ethyl acetate layer generated by the fractionation of the *Magnolia flower* extract with ethyl acetate;
2) obtaining an eluate by adding the mixed solvent comprising ethyl acetate and hexane to the methanol fraction of step 1); and
3) precipitating crystals in the eluate of step 2), followed by filtering thereof with a filter.

16. The method for isolating the compound represented by formula 1 or formula 2 according to claim 15, wherein the *Magnolia flower* extract is extracted by using water, C₁ ∼ C₂ lower alcohol, or a mixed solvent thereof as an extraction solvent.

17. The method for isolating the compound represented by formula 1 or formula 2 according to claim 15, wherein the mixed solvent of step 2 contains ethyl acetate and hexane at the ratio of 3:7 or 5:5.

18. A method for accelerating the growth of dental root or periodontal tissue, or preventing or treating periodontitis comprising the step of administering a pharmaceutically effective dose of a *Magnolia flower* extract or a fraction isolated from the same to a subject.

19. A use of the pharmaceutical composition comprising a *Magnolia flower* extract or a fraction isolated therefrom as an active ingredient for accelerating the growth of dental root or periodontal tissue, or preventing or treating periodontitis.

20. A use of the quasi-drug composition comprising a *Magnolia flower* extract or a fraction isolated therefrom as an active ingredient for accelerating the growth of dental root or periodontal tissue, or preventing or improving periodontitis.

21. A use of the health functional food comprising a *Magnolia flower* extract or a fraction isolated therefrom as an active ingredient for accelerating the growth of dental root or periodontal tissue, or preventing or improving periodontitis.

22. A use of the cosmetic composition comprising a *Magnolia flower* extract or a fraction isolated therefrom as an active ingredient for accelerating the growth of dental root or periodontal tissue, or improving periodontitis.

23. A method for accelerating the growth of dental root or periodontal tissue, or preventing or treating periodontitis comprising the step of administering a pharmaceutically effective dose of the compound represented by formula 1 or formula 2, the pharmaceutically acceptable salt thereof, or the optical isomer thereof to a subject.

24. A use of the pharmaceutical composition comprising the compound represented by formula 1 or formula 2, the pharmaceutically acceptable salt thereof, or the optical isomer thereof as an active ingredient for accelerating the growth of dental root or periodontal tissue, or preventing or treating periodontitis.

25. A use of the quasi-drug composition comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

26. A use of the health functional food comprising the compound represented by formula 1 or formula 2, the pharmaceutically acceptable salt thereof, or the optical isomer thereof as an active ingredient for accelerating the growth of dental root or periodontal tissue, or preventing or improving periodontitis.

27. A use of the cosmetic composition comprising the compound represented by formula 1 or formula 2, the pharmaceutically acceptable salt thereof, or the optical isomer thereof as an active ingredient for accelerating the growth of dental root or periodontal tissue, or improving periodontitis.
